(19) Europäisches Patentamt
European Patent Office
Office européen des brevets

(11) EP 4 652 993 A1

(12) EUROPEAN PATENT APPLICATION
published in accordance with Art. 153(4) EPC

(43) Date of publication:
**26.11.2025 Bulletin 2025/48**

(21) Application number: **24744196.7**

(22) Date of filing: **15.01.2024**

(51) International Patent Classification (IPC):
***A61K 31/407*** (2006.01) ***A61K 31/427*** (2006.01)
***A61K 9/48*** (2006.01) ***A61K 9/28*** (2006.01)
***A61K 9/20*** (2006.01) ***A61K 9/16*** (2006.01)
***A61K 9/107*** (2006.01) ***A61K 9/08*** (2006.01)
***A61K 47/38*** (2006.01) ***A61K 47/26*** (2006.01)
***A61K 47/04*** (2006.01) ***A61K 47/02*** (2006.01)
***A61P 31/14*** (2006.01)

(52) Cooperative Patent Classification (CPC):
**A61K 9/08; A61K 9/107; A61K 9/16; A61K 9/20; A61K 9/28; A61K 9/48; A61K 31/407; A61K 31/427; A61K 47/02; A61K 47/26; A61K 47/38; A61P 31/14**

(86) International application number:
**PCT/CN2024/072261**

(87) International publication number:
**WO 2024/153020 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.01.2023 CN 202310081244**

(71) Applicant: **Hainan Simcere Pharmaceutical Co., Ltd.**
**Haikou, Hainan 570311 (CN)**

(72) Inventors:
- **PENG, Tao**
  **Nanjing, Jiangsu 210042 (CN)**
- **TAN, Ying**
  **Nanjing, Jiangsu 210042 (CN)**
- **LEI, Xiaoxue**
  **Nanjing, Jiangsu 210042 (CN)**
- **ZHANG, Lei**
  **Nanjing, Jiangsu 210042 (CN)**

(74) Representative: **Mewburn Ellis LLP**
**Aurora Building**
**Counterslip**
**Bristol BS1 6BX (GB)**

# (54) ANTIVIRAL PHARMACEUTICAL COMPOSITION AND PREPARATION METHOD THEREFOR

(57) Provided are an oral pharmaceutical composition containing a spiro compound as represented by formula (I) or a pharmaceutically acceptable salt thereof, a method for preparing the oral pharmaceutical composition, a combined product containing the oral pharmaceutical composition and another antiviral drug, and the use of the oral pharmaceutical composition and the combined product against viral infections and in the treatment or prevention of related diseases caused by viral infections.

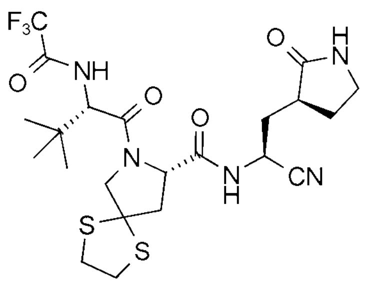

(I)

EP 4 652 993 A1

**Description**

## CROSS-REFERENCE TO RELATED APPLICATION

The present application claims the benefit of and priority to the Chinese Patent Application No. 202310081244.6 filed with China National Intellectual Property Administration on January 16, 2023, which is incorporated herein by reference in its entirety.

## TECHNICAL FIELD

The present disclosure relates to the field of pharmaceutical formulations, and particularly relates to an oral pharmaceutical composition, a preparation method therefor, and use thereof in resisting viral infection.

## BACKGROUND

Coronaviruses are single-stranded positive-sense RNA viruses. Some coronaviruses can spread widely in the human population and can cause severe symptoms. There are 7 coronaviruses known to be capable of infecting humans, namely HCoV-229E, HCoV-OC43, HCoV-NL63, HCoV-HKU1, SARS-CoV, MERS-CoV, and SARS-CoV-2. Most functional proteins of coronaviruses are encoded by ORF1ab genes, and they are first translated into a polyprotein and then cleaved by 3CL protease and PL protease into multiple active proteins. Therefore, inhibiting the activity of 3CL protease can effectively inhibit the replication of the viruses. Different coronavirus 3CL proteases have high structural homology. Therefore, in general, 3CL protease inhibitors have broad-spectrum anti-coronavirus activity.

In addition to coronaviruses, 3CL protease also plays an important role in the proteolysis of polyproteins encoded by picornaviruses. 3CL protease inhibitors can effectively inhibit the replication of picornaviruses. Enterovirus 71 is a picornavirus, which is one of the common viruses causing hand, foot and mouth disease, and may also cause various diseases such as meningitis, brainstem encephalitis, myocarditis, and the like. In recent years, enterovirus 71 has exploded many times in the population of infants and young children, and there is still a lack of efficient therapeutic drugs clinically.

Therefore, there is still a clinical need for antiviral pharmaceutical formulations for treating coronaviruses, enterovirus 71, etc.

## SUMMARY

In one aspect, the present disclosure provides an oral pharmaceutical composition, comprising a compound of formula (I) or a pharmaceutically acceptable salt thereof:

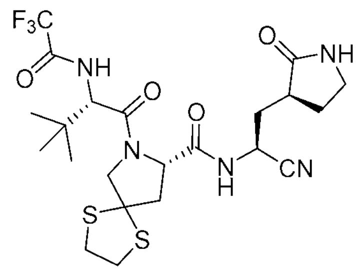

I .

In another aspect, the present disclosure provides a preparation method for the oral pharmaceutical composition, comprising: (1) mixing the compound of formula (I) or the pharmaceutically acceptable salt thereof with one or more of a disintegrant, a filler, a glidant, and a lubricant; and (2) optionally, coating the mixture obtained in step (1) with a coating agent.

In another aspect, the present disclosure further provides a combination product, comprising: (1) the oral pharmaceutical composition, and (2) an additional antiviral drug.

In another aspect, the present disclosure further provides use of the oral pharmaceutical composition or the combination product in the preparation of a medicament for preventing or treating a related disease caused by infection with a coronavirus and/or a picornavirus.

In another aspect, the present disclosure further provides use of the oral pharmaceutical composition or the combination product in the prevention or treatment of a related disease caused by infection with a coronavirus and/or a

picornavirus.

In another aspect, the present disclosure further provides the oral pharmaceutical composition or the combination product for use in the prevention or treatment of a related disease caused by infection with a coronavirus and/or a picornavirus.

In another aspect, the present disclosure provides a method for treating a related disease caused by infection with a coronavirus and/or a picornavirus, comprising administering to a mammal, preferably a human, in need of the treatment a therapeutically effective amount of the oral pharmaceutical composition or the combination product.

## DETAILED DESCRIPTION

In one aspect, the present disclosure provides an oral pharmaceutical composition, comprising a compound of formula (I) or a pharmaceutically acceptable salt thereof:

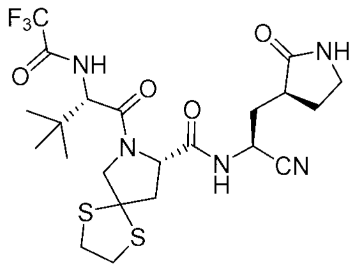

I .

In some embodiments, the oral pharmaceutical composition is in the form of a tablet, a capsule, a pill, a granule, a powder, an emulsion, a solution, or a suspension.

In some embodiments, the oral pharmaceutical composition is in the form of a tablet, a capsule, a granule, a powder, or a suspension.

In some embodiments, the oral pharmaceutical composition is a tablet.

In some embodiments, the oral pharmaceutical composition further comprises a disintegrant.

In some embodiments, the disintegrant is selected from one or more of low-substituted hydroxypropyl cellulose, carboxymethylcellulose calcium, crospovidone, dry starch, sodium carboxymethyl starch, and croscarmellose sodium.

In some embodiments, the disintegrant is selected from one or more of low-substituted hydroxypropyl cellulose, crospovidone, and croscarmellose sodium.

In some embodiments, the disintegrant is croscarmellose sodium.

In some embodiments, the oral pharmaceutical composition further comprises a filler.

In some embodiments, the filler is selected from one or more of microcrystalline cellulose, calcium sulfate, calcium hydrogen phosphate, calcium carbonate, calcium sulfate dihydrate, lactose, sucrose, dextrin, sorbitol, starch or a derivative thereof, mannitol, xylitol, and fructose.

In some embodiments, the filler is selected from one or more of microcrystalline cellulose, lactose, dextrin, and starch.

In some embodiments, the filler is selected from one or more of microcrystalline cellulose and lactose. In some embodiments, the filler is microcrystalline cellulose and lactose.

In some embodiments, the lactose is lactose monohydrate.

In some embodiments, the oral pharmaceutical composition further comprises a glidant.

In some embodiments, the glidant is selected from one or more of colloidal silicon dioxide, talc, and wheat starch.

In some embodiments, the glidant is selected from one or more of colloidal silicon dioxide and talc. In some embodiments, the glidant is colloidal silicon dioxide.

In some embodiments, the oral pharmaceutical composition further comprises a lubricant.

In some embodiments, the lubricant is selected from one or more of magnesium stearate, stearic acid, calcium stearate, zinc stearate, sodium stearyl fumarate, liquid paraffin, polyethylene glycol, sodium dodecyl sulfate, and hydrogenated vegetable oil.

In some embodiments, the lubricant is selected from one or more of magnesium stearate, stearic acid, calcium stearate, and sodium stearyl fumarate.

In some embodiments, the lubricant is sodium stearyl fumarate.

In some embodiments, the present disclosure provides an oral pharmaceutical composition, comprising the compound of formula (I) or the pharmaceutically acceptable salt thereof, a disintegrant, a filler, a glidant, and a lubricant.

In some embodiments, the present disclosure provides an oral pharmaceutical composition, comprising the

compound of formula (I) or the pharmaceutically acceptable salt thereof, a disintegrant, a filler, a glidant, and a lubricant, wherein the disintegrant is croscarmellose sodium, the filler is microcrystalline cellulose and lactose monohydrate, the glidant is colloidal silicon dioxide, and the lubricant is sodium stearyl fumarate.

In some embodiments, the oral pharmaceutical composition comprises the compound of formula (I) or the pharmaceutically acceptable salt thereof, a disintegrant, a filler, a glidant, and a lubricant, wherein in parts by weight, in the oral pharmaceutical composition, the content of the compound of formula (I) or the pharmaceutically acceptable salt thereof is 20-80 parts, the content of the disintegrant is 0.5-20 parts, the content of the filler is 10-70 parts, the content of the glidant is 0.1-20 parts, and the content of the lubricant is 0.1-20 parts.

In some embodiments, in parts by weight, in the oral pharmaceutical composition, the content of the compound of formula (I) or the pharmaceutically acceptable salt thereof is 20-80 parts, the content of croscarmellose sodium is 0.5-20 parts, the total content of microcrystalline cellulose and lactose monohydrate is 10-70 parts, the content of colloidal silicon dioxide is 0.1-20 parts, and the content of sodium stearyl fumarate is 0.1-20 parts.

In some embodiments, in parts by weight, in the oral pharmaceutical composition, the content of the compound of formula (I) or the pharmaceutically acceptable salt thereof is 45-55 parts.

In some embodiments, in parts by weight, in the oral pharmaceutical composition, the content of the filler is 30-45 parts.

In some embodiments, in parts by weight, in the oral pharmaceutical composition, the content of the disintegrant is 2-10 parts.

In some embodiments, in parts by weight, in the oral pharmaceutical composition, the content of the glidant is 1-3 parts.

In some embodiments, in parts by weight, in the oral pharmaceutical composition, the content of the lubricant is 1-3 parts.

In some embodiments, in parts by weight, in the oral pharmaceutical composition, the content of the compound of formula (I) or the pharmaceutically acceptable salt thereof is 45-55 parts, the content of the disintegrant is 2-10 parts, the content of the filler is 30-45 parts, the content of the glidant is 1-3 parts, and the content of the lubricant is 1-3 parts.

In some embodiments, in parts by weight, in the oral pharmaceutical composition, the content of the compound of formula (I) or the pharmaceutically acceptable salt thereof is 45-55 parts, the content of croscarmellose sodium is 2-10 parts, the total content of microcrystalline cellulose and lactose monohydrate is 30-45 parts, the content of colloidal silicon dioxide is 1-3 parts, and the content of sodium stearyl fumarate is 1-3 parts.

In another aspect, the present disclosure provides an oral pharmaceutical composition, comprising the compound of formula (I) or the pharmaceutically acceptable salt thereof described above, a disintegrant, a filler, a glidant, a lubricant, and a coating agent.

In some embodiments, the coating agent is a gastric-soluble film coating agent.

In some embodiments, the coating agent comprises hydroxypropyl methylcellulose, iron oxide red, polyethylene glycol, and titanium dioxide.

In some embodiments, the present disclosure provides an oral pharmaceutical composition, comprising the compound of formula (I) or the pharmaceutically acceptable salt thereof, a disintegrant, a filler, a glidant, a lubricant, and a coating agent, wherein the disintegrant is croscarmellose sodium, the filler is microcrystalline cellulose and lactose monohydrate, the glidant is colloidal silicon dioxide, the lubricant is sodium stearyl fumarate, the coating agent comprises hydroxypropyl methylcellulose, iron oxide red, polyethylene glycol, and titanium dioxide.

In some embodiments, in parts by weight, in the oral pharmaceutical composition, the content of the coating agent is 0.5-20 parts.

In some embodiments, in parts by weight, in the oral pharmaceutical composition, the content of the coating agent is 2-6 parts.

In some embodiments, in parts by weight, in the oral pharmaceutical composition, the content of the compound of formula (I) or the pharmaceutically acceptable salt thereof is 20-80 parts, the content of the disintegrant is 0.5-20 parts, the content of the filler is 10-70 parts, the content of the glidant is 0.1-20 parts, the content of the lubricant is 0.1-20 parts, and the content of the coating agent is 0.5-20 parts.

In some embodiments, in parts by weight, in the oral pharmaceutical composition, the content of the compound of formula (I) or the pharmaceutically acceptable salt thereof is 45-55 parts, the content of the disintegrant is 2-10 parts, the content of the filler is 30-45 parts, the content of the glidant is 1-3 parts, the content of the lubricant is 1-3 parts, and the content of the coating agent is 2-6 parts.

In some embodiments, in parts by weight, in the oral pharmaceutical composition, the content of the compound of formula (I) or the pharmaceutically acceptable salt thereof is 20-80 parts, the content of croscarmellose sodium is 0.5-20 parts, the total content of microcrystalline cellulose and lactose monohydrate is 10-70 parts, the content of colloidal silicon dioxide is 0.1-20 parts, the content of sodium stearyl fumarate is 0.1-20 parts, and the content of the coating agent comprising hydroxypropyl methylcellulose, iron oxide red, polyethylene glycol, and titanium dioxide is 0.5-20 parts.

In some embodiments, in parts by weight, in the oral pharmaceutical composition, the content of the compound of

formula (I) or the pharmaceutically acceptable salt thereof is 45-55 parts, the content of croscarmellose sodium is 2-10 parts, the total content of microcrystalline cellulose and lactose monohydrate is 30-45 parts, the content of colloidal silicon dioxide is 1-3 parts, the content of sodium stearyl fumarate is 1-3 parts, and the content of the coating agent comprising hydroxypropyl methylcellulose, iron oxide red, polyethylene glycol, and titanium dioxide is 2-6 parts.

In some embodiments, in the oral pharmaceutical composition, the content of the compound of formula (I) or the pharmaceutically acceptable salt thereof is 10 mg-1000 mg, e.g., 10 mg, 50 mg, 100 mg, 150 mg, 200 mg, 250 mg, 300 mg, 350 mg, 375 mg, 400 mg, 450 mg, 500 mg, 550 mg, 600 mg, 650 mg, 700 mg, 750 mg, 800 mg, 850 mg, 900 mg, 950 mg, or 1000 mg.

In some embodiments, when the oral pharmaceutical composition is a tablet, the content of the compound of formula (I) or the pharmaceutically acceptable salt thereof is 50 mg/tablet, 150 mg/tablet, 375 mg/tablet, or 500 mg/tablet.

In another aspect, the present disclosure provides a preparation method for the oral pharmaceutical composition, comprising: (1) mixing the compound of formula (I) or the pharmaceutically acceptable salt thereof with one or more of a disintegrant, a filler, a glidant, and a lubricant; and (2) optionally, coating the mixture obtained in step (1) with a coating agent.

In another aspect, the present disclosure further provides a combination product, comprising: (1) the oral pharmaceutical composition, and (2) an additional antiviral drug.

In some embodiments, the additional antiviral drug is an anti-coronavirus and/or picornavirus drug. In some embodiments, the additional antiviral drug is ritonavir.

In another aspect, the present disclosure further provides use of the oral pharmaceutical composition or the combination product in the preparation of a medicament for preventing or treating a related disease caused by infection with a coronavirus and/or a picornavirus.

In another aspect, the present disclosure further provides use of the oral pharmaceutical composition or the combination product in the prevention or treatment of a related disease caused by infection with a coronavirus and/or a picornavirus.

In another aspect, the present disclosure further provides the oral pharmaceutical composition or the combination product for use in the prevention or treatment of a related disease caused by infection with a coronavirus and/or a picornavirus.

In another aspect, the present disclosure provides a method for treating a related disease caused by infection with a coronavirus and/or a picornavirus, comprising administering to a mammal, preferably a human, in need of the treatment a therapeutically effective amount of the oral pharmaceutical composition or the combination product.

In some embodiments, the related disease caused by infection with the coronavirus and/or the picornavirus described herein includes, but is not limited to, respiratory tract infection, pneumonia, or a complication thereof.

In some embodiments, the coronavirus described herein is selected from SARS-CoV, MERS-CoV, H229E-CoV, HKU1-CoV, NL63-CoV, OC43-CoV, and SARS-CoV-2.

In some embodiments, examples of the picornavirus described herein include, but are not limited to, enterovirus type 71.

## BRIEF DESCRIPTION OF THE DRAWINGS

FIG. 1 shows the inhibitory effects of a compound of formula (I) on the viral titer in the lungs of mice 2 days (panel A) and 4 days (panel B) after infection in Test Example 4.

FIG. 2 shows the changes in body weight of mice in Test Example 4.

FIG. 3 shows the inhibitory effect of a compound of formula (I) on the viral titer in the brains of mice 4 days after infection in Test Example 4.

FIG. 4 shows the *in vitro* dissolution release curves of the coated tablet in Test Example 11.

## Examples

The present disclosure is described in detail below by way of examples, which, however, are not intended to disadvantageously limit the scope of the present disclosure in any way. Although the present disclosure has been described in detail herein and specific embodiments thereof have also been disclosed, it will be apparent to those skilled in the art that various changes and modifications can be made to the specific embodiments of the present disclosure without departing from the spirit and scope of the present disclosure. All reagents used in the present disclosure are commercially available and can be used without further purification.

Unless otherwise stated, the ratios expressed for mixed solvents are volume mixing ratios. Unless otherwise stated, % refers to wt%.

Compounds are named either manually or by ChmDraw® software, and supplier's catalog names are given for commercially available compounds.

The structures of the compounds are determined by nuclear magnetic resonance (NMR) and/or mass spectrometry (MS). NMR shifts are given in $10^{-6}$ (ppm). The solvents for NMR determination are deuterated dimethyl sulfoxide, deuterated chloroform, deuterated methanol, and the like, and the internal standard is tetramethylsilane (TMS).

**Example 1: Preparation of Compound of Formula (I)**

1.1. Preparation of compound 1-1:

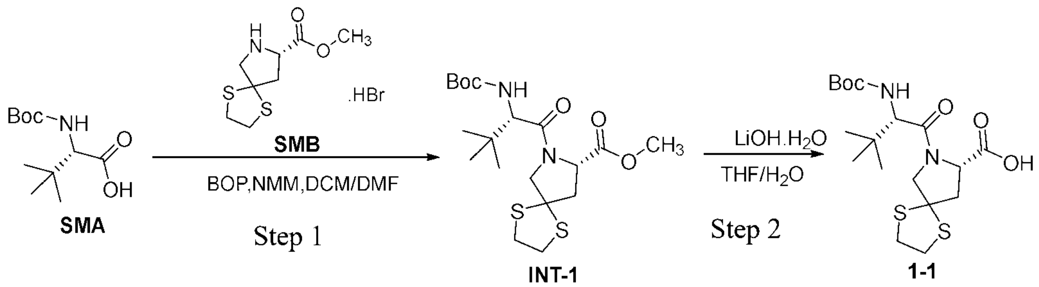


Step 1: Starting material SMA (2.74 g, 11.85 mmol), 35 mL of dichloromethane, and 35 mL of DMF were added to a reaction flask. The mixture was cooled to 0 °C. Starting material SMB (3.56 g, 11.86 mmol), benzotriazol-1-yloxytris(dimethylamino)phosphonium hexafluorophosphate (BOP, 6.29 g, 14.22 mmol), and *N*-methylmorpholine (NMM, 3.91 mL, 35.56 mmol) were sequentially added. The mixture was heated to room temperature and allowed to react for 10 h. After the reaction was completed, a proper amount of dichloromethane was added. The organic phase was washed sequentially with a 1 N aqueous hydrochloric acid solution and saturated brine. After the washing was completed, the organic phase was dried over anhydrous sodium sulfate and concentrated to dryness, and the residue was subjected to column chromatography to give 3.71 g of INT-1; ESI-MS: 433.2 m/z $[M+H]^+$; $^1H$ NMR (400 MHz, DMSO-$d_6$): $\delta_H$: 6.75 (d, J = 9.2 Hz, 1H), 4.38 (t, J = 8.2 Hz, 1H), 4.25 (d, J = 10.9 Hz, 1H), 4.11 (d, J = 9.3 Hz, 1H), 3.93 (t, J = 9.3 Hz, 1H), 3.62 (s, 3H), 3.40-3.31 (m, 4H), 2.70 (dd, J = 13.1, 7.9 Hz, 1H), 2.37 (dd, J = 13.2, 8.4 Hz, 1H), 1.37 (s, 9H), 0.94 (s, 9H).

Step 2: INT-1 (3.71 g, 8.58 mmol), 37 mL of THF, 37 mL of purified water, and lithium hydroxide monohydrate (0.72 g, 17.16 mmol) were added to a reaction flask, and the mixture was allowed to react at room temperature for 2 h. After the reaction was completed, the mixture was adjusted to pH 4 with concentrated hydrochloric acid and filtered to give 3.4 g of compound 1-1; ESI-MS: 419.2 m/z $[M+H]^+$; $^1H$ NMR (400 MHz, DMSO-$d_6$): $\delta_H$:12.68 (s, 1H), 6.71 (d, J = 9.4 Hz, 1H), 4.38-4.19 (m, 2H), 4.11 (d, J = 9.4 Hz, 1H), 3.88 (d, J = 10.9 Hz, 1H), 3.41-3.29(m, 4H), 2.69 (dd, J = 13.1, 7.9 Hz, 1H), 2.34 (dd, J = 13.2, 8.9 Hz, 1H), 1.38 (s, 9H), 0.94 (s, 9H).

1.2. Preparation of compound 1-2:

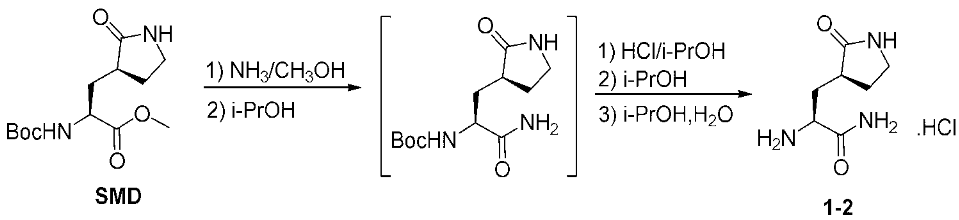


A solution of ammonia in methanol (700 mL, 7 mol/L) and starting material SMD (100 g, 0.349 mol) were added to a reaction flask and stirred until the solid was completely dissolved. The mixture was allowed to react for 36 h with the temperature maintained at 25±5 °C. After the reaction was completed, the reaction mixture was concentrated until the residual reaction mixture was about 250 mL, then 300 mL of isopropanol was added, and concentration under reduced pressure was continued until the residual reaction mixture was about 250 mL (this procedure was repeated three times). The system was purged with nitrogen and cooled to 10±5 °C. 500 mL of a solution of hydrogen chloride in isopropanol (4 mol/L) was added to the reaction kettle, and after the addition was completed, the mixture was heated to 25±5 °C and

allowed to react for 9 h with the temperature maintained at 25±5 °C. After the reaction was completed, the reaction mixture was concentrated under reduced pressure until the residual volume of the reaction mixture was about 250 mL, then 300 mL of isopropanol was added, and concentration under reduced pressure was continued until the residual volume of the reaction mixture was about 250 mL (this procedure was repeated twice). Then, 100 mL of isopropanol was added, and the mixture was stirred for 30±5 min and filtered. The filter cake was rinsed with 50 mL of isopropanol to give a wet product, which was then dried under vacuum at 45±5 °C to give 66.7 g of compound 1-2; $^1$H NMR (400 MHz, DMSO-$d_6$): $\delta_H$:8.45 (d, J = 5.1 Hz, 3H), 8.25-8.04 (m, 1H), 7.95 (s, 1H), 7.67-7.49 (m, 1H), 3.85-3.80 (m, 1H), 3.19-3.13 (m, 2H), 2.59-2.51 (m, 1H), 2.32-2.27 (m, 1H), 2.05-1.98 (m, 1H), 1.82-1.66 (m, 2H); ESI-MS: 172.1 m/z [M+H]$^+$. 1.3. Preparation of compound of formula (I):

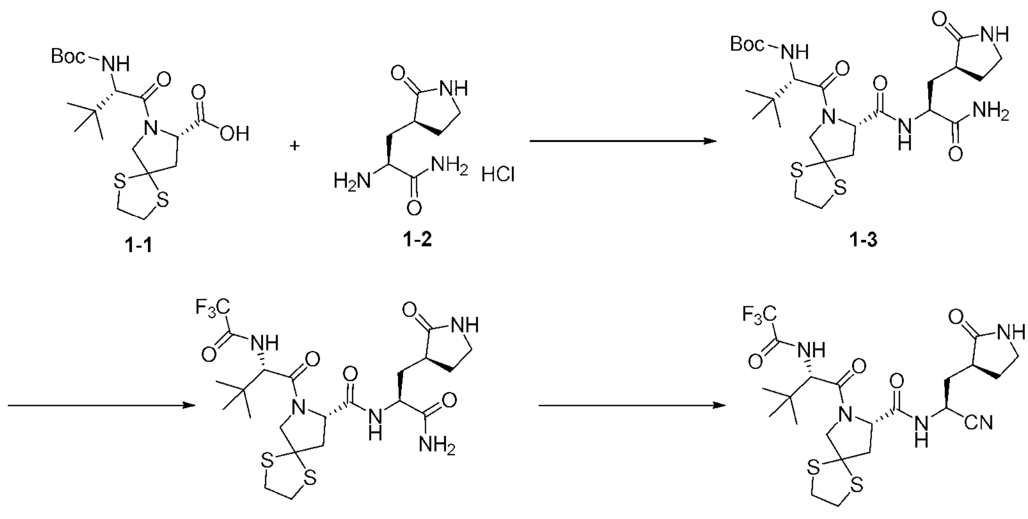

1-1 1-2 1-3

1-4 I

Step 1: Compound 1-1 (419 mg, 1 mmol) was placed in a double-necked flask, 5 mL of dichloromethane was added under nitrogen atmosphere, then *O*-(7-azabenzotriazol-1-yl)-*N,N,N',N'*-tetramethyluronium hexafluorophosphate (400 mg, 1.1 mmol) was added, and the reaction mixture was stirred at room temperature for 1 h. Compound 1-2 (1 mmol) was dissolved in 1 mL of dichloromethane, and the mixture was added to the above system. Subsequently, *N,N*-diisopropylethylamine (2 mmol) was added under an ice-water bath. The ice-water bath was then removed, and the system was stirred at room temperature overnight. For post-treatment: 50 mL of dichloromethane was added, and the mixture was washed three times with a 1 M aqueous hydrochloric acid solution and then washed three times with a saturated aqueous sodium bicarbonate solution. The organic phase was washed with saturated brine, dried over anhydrous sodium sulfate, filtered, and concentrated to dryness by rotary evaporation to give compound 1-3 (469 mg). ESI-MS: m/z 572.3[M+H]$^+$.

Step 2: Compound 1-3 (572 mg, 1 mmol) was dissolved in 3 mL of a 4 M solution of hydrogen chloride in 1,4-dioxane, and the mixture was stirred at ambient temperature. After the starting materials were substantially consumed as detected by thin layer chromatography, the mixture was concentrated to dryness by rotary evaporation to remove the solvent. The resulting crude product was dissolved in 2 mL of dichloromethane, and triethylamine (3 mmol) was added under nitrogen atmosphere. The system was placed in an ice-water bath, and trifluoroacetic anhydride (1.2 mmol) was added dropwise. After the starting materials were substantially consumed as detected by thin layer chromatography, 50 mL of dichloromethane was added, and the mixture was washed three times with a 1 M aqueous hydrochloric acid solution and then washed three times with a saturated aqueous sodium bicarbonate solution. The organic phase was washed with saturated brine, dried over anhydrous sodium sulfate, and subjected to column chromatography to give compound 1-4 (265 mg). ESI-MS: m/z 568.3[M+H]$^+$.

Step 3: Compound 1-4 (113 mg, 0.2 mmol) and Burgess reagent (1.5 eq) were added to a two-necked flask. The system was purged with nitrogen three times, and then dichloromethane dried with molecular sieves was added. The mixture was stirred at room temperature overnight, and the starting materials were substantially consumed as detected by thin layer chromatography. For post-treatment: The mixture was subjected to column chromatography to give a compound of formula (I) (41 mg). $^1$H NMR (400 MHz, DMSO-$d_6$) $\delta$ 9.46 (d, *J* = 8.7 Hz, 1H), 9.05 (d, *J* = 8.6 Hz, 1H), 7.67 (s, 1H), 4.97 (ddd, *J* = 11.0, 8.5, 5.0 Hz, 1H), 4.53 (d, *J* = 8.7 Hz, 1H), 4.34 (dd, *J* = 9.9, 7.1 Hz, 1H), 4.26-4.14 (m, 1H), 3.92 (d, *J* = 10.9 Hz, 1H), 3.50-3.34 (m, 4H), 3.22-3.11 (m, 1H), 3.06 (td, *J* = 9.3, 7.1 Hz, 1H), 2.68-2.58 (m, 1H), 2.50-2.43 (m, 1H), 2.31 (dd, *J* = 13.0, 10.0 Hz, 1H), 2.23-2.07 (m, 2H), 1.71 (tdd, *J* = 14.9, 10.3, 7.4 Hz, 2H), 0.99 (s, 9H). ESI-MS: 550.3 m/z [M+H]$^+$.

**Example 2: Preparation of Coated Tablets**

(1) About 50 g of the compound of formula (I) was weighed and pulverized using a jet mill with a particle size standard: d (0.9) of 5-20 μm. The corresponding excipients were weighed according to the formula amounts in Table 1.
(2) The weighed microcrystalline cellulose (intragranular addition), croscarmellose sodium (intragranular addition), colloidal silicon dioxide (intragranular addition), pulverized compound of formula (I) (intragranular addition), and lactose monohydrate (intragranular addition) were sequentially transferred into a mixing bucket for mixing (rotation speed: 10 rpm, mixing time: 10 min) to give a premix 1.
(3) Sodium stearyl fumarate (intragranular addition) was added into the premix 1, and mixing was continued (rotation speed: 10 rpm, mixing time: 3 min) to give a premix 2.
(4) The premix 2 was sieved using a granulator (pore size: φ 0.8 mm). After sieving, the material was added into the above mixing bucket for mixing (rotation speed: 10 rpm, mixing time: 5 min) to give a premix 3.
(5) The premix 3 was subjected to dry granulation to give granules.
(6) The prepared granules, colloidal silicon dioxide (extragranular addition), and croscarmellose sodium (extragranular addition) were added into the mixing bucket for mixing (rotation speed: 10 rpm, mixing time: 5 min) to give a final mixture 1.
(7) Sodium stearyl fumarate (extragranular addition) was added to the above final mixture 1, and mixing was continued (rotation speed: 10 rpm, mixing time: 3 min) to give a final mixture 2.
(8) The final mixture 2 was compressed into tables. The theoretical tablet weights were 0.1 g, 0.3 g, 0.75 g, and 1 g as shown in Table 1, and the hardness was 110-280 N.
(9) Preparation of a coating solution at a concentration of about 12% (w/w): Purified water was added, and when the purified water was stirred into a vortex, a gastric-soluble film coating premix (Colorcon, with ingredients including: hydroxypropyl methylcellulose, iron oxide red, polyethylene glycol, and titanium dioxide) was added to the vortex shoulder of the vortex. The mixture was stirred for 1 h to form a homogeneous suspension, and then stirring was suspended. The coating solution was then sieved through a 60-mesh sieve, and it was confirmed that there was no residue on the sieve, thereby completing the preparation of the coating solution.
(10) Coating: The compressed tablets obtained in step (8) were coated with the coating solution. The entire coating process involved three steps: dust removal and preheating, coating, and cooling. The coating weight gain was controlled at 4%.

**Table 1. Formula composition for tablets**

| | Ingredient | | Amount (mg/tablet) | | | | |
|---|---|---|---|---|---|---|---|
| | | | F1 | F2 | F3 | F4 | % (w/w) |
| Tablet core | Intragranular addition material | Compound of formula (I) | 50 | 150 | 375 | 500 | 50% |
| | | Microcrystalline cellulose | 30 | 90 | 225 | 300 | 30% |
| | | Lactose monohydrate | 11 | 33 | 82.5 | 110 | 11% |
| | | Croscarmellose sodium | 2 | 6 | 15 | 20 | 2% |
| | | Colloidal silicon dioxide | 1 | 3 | 7.5 | 10 | 1% |
| | | Sodium stearyl fumarate | 1 | 3 | 7.5 | 10 | 1% |

(continued)

| | Ingredient | | Amount (mg/tablet) | | | | |
|---|---|---|---|---|---|---|---|
| | | | F1 | F2 | F3 | F4 | % (w/w) |
| | Extragranular addition material | Croscarmellose sodium | 3 | 9 | 22.5 | 30 | 3% |
| | | Colloidal silicon dioxide | 1 | 3 | 7.5 | 10 | 1% |
| | | Sodium stearyl fumarate | 1 | 3 | 7.5 | 10 | 1% |
| Total weight | | | 100 | 300 | 750 | 1000 | 100% |
| Coating | Film coating premix (gastric-soluble) | | 4 | 12 | 30 | 40 | 4% |
| | Purified water | | Proper amount | Proper amount | Proper amount | Proper amount | / |

**Test Examples for Biological Activity and Related Properties**

**Test Example** 1-1: Test of Inhibitory Activity against SARS-CoV-2 3CLpro by Compound of Formula (I)

The inhibitory activity of the compound of formula (I) against enzymatic activity of SARS-CoV-2 $3CL^{pro}$ was evaluated by fluorescence resonance energy transfer. The volume of the entire enzymatic reaction system was 120 μL, the final concentration of the protease was 30 nM, and the final concentration of the substrate was 20 μM. The buffer of the reaction system included 50 mM Tris pH 7.3 and 1 mM EDTA. SARS-CoV-2 $3CL^{pro}$ protease and compounds at different concentrations were added into a 96-well plate, and the plate was incubated at 30 °C for 10 min. The substrate was then added, and the plate was promptly placed in a microplate reader for reading. The excitation light and the emission light were 320 nM and 405 nM, respectively. The test time was 3.5 min, and fluorescence values were read every 35 s. The reaction rates were obtained by fitting the readings of the first 2 min of the final results and were compared with that of the control group (DMSO), and the inhibition rates were calculated. The $IC_{50}$ values and inhibition rate curves were obtained by fitting using GraphPad Prism 8 software.

The experimental results showed that the compound of formula (I) exhibited a potent inhibitory effect on SARS-CoV-2 $3CL^{pro}$, with an $IC_{50}$ value of < 0.1 μM.

**Test Example 1-2: Test of Inhibitory Activity against Mutant 3CL Protease in SARS-CoV-2 Omicron Strain by Compound of Formula (I)**

Experimental principle: The inhibitory effect of the compound of the present disclosure on the activity of the mutant 3CL protease (P132H) in the Omicron strain was investigated by using a method in which an enzyme reacts with a substrate to generate fluorescence resonance energy transfer (FRET). The experimental materials are shown in the table below:

| Reagent material | Brand | Catalog No. |
|---|---|---|
| 3CL Protease-P132H mutant | Shanghai Panchao | / |
| Dabcyl-KTSAVLQSGFRKME-Edans (Coronavirus main protease fluorescent substrate) | Beyotime | P9733 |
| DTT (dithiothreitol) | Invitrogen | P2325 |
| BSA (bovine serum albumin) | Sigma | V900933 |
| EDTA (ethylenediaminetetraacetic acid) | Invitrogen | AM9260G |
| Tris-HCl (tris(hydroxymethyl)aminomethane) | Sangon Biotech | B548127 |

Experimental instruments and equipment:

| Instrument | Brand | Model |
|---|---|---|
| Echo nano-liter scale acoustic liquid handling system | Labcyte | Echo 650 |
| Flexstation 3 microplate reader | MolecMar Devices | FLEX3 |
| Centrifuge | Eppendorf | 5810 |

Experimental procedures:

A reaction buffer containing 20 mM Tris-HCl, 1 mM EDTA, 0.01% BSA, 1 mM DTT, and 100 mM NaCl was prepared. The test compounds were diluted to different concentrations in dimethyl sulfoxide (DMSO) using the Echo liquid handling system and transferred to a 384-well plate. The mutant 3CL protease was diluted with a reaction buffer and added to the 384-well plate at 10 μL/well. The plate was centrifuged at 1000 rpm for 1 min and then incubated at room temperature for 30 min.

A substrate was then added at 10 μL/well, and the plate was centrifuged at 1000 rpm for 30 s to start the enzymatic reaction. In the reaction system, the final concentration of the enzyme was 50 nM, the final concentration of the substrate was 20 μM, and the concentration range of the compound was 10000 nM to 0.51 nM. Then the Kinetic Reduction Vmax mode was selected on the Flexstation 3 microplate reader, and the fluorescence values at the wavelength of 490 nm were continuously read every 75 s for 35 times to obtain the reaction rate values (V). The inhibition rates were calculated, and the half maximal inhibitory concentrations ($IC_{50}$) were obtained by four-parameter fitting using XLfit software. The calculation method of inhibition rate is as follows:

$$\text{Inhibition rate} = (V_{max} - V_{compound})/(V_{max} - V_{min}) \times 100\%$$

$V_{max}$ is the reaction rate value of wells containing only enzyme and substrate, $V_{min}$ is the reaction rate value of wells containing only substrate, and $V_{Compound}$ is the reaction rate value of wells containing test compound, enzyme and substrate.

Experimental results: The compound of formula (I) retained significant inhibitory activity against the 3CL protease having a P132H mutation in SARS-CoV-2 Omicron strain.

**Table 2. Inhibitory effect of compound of formula (I) on activity of 3CL protease in SARS-CoV-2 Omicron strain**

| 3CL protease | Compound of formula (I) $IC_{50}$ (μM) (n = 3*) |
|---|---|
| SARS-CoV-2 (Omicron strain) | 0.022±0.00090 |
| * Refers to three independent replicates. | |

**Test Example 2: Test of Inhibitory Activity against Coronavirus 3CL Protease of Different Sources by Compound of Formula (I)**

Experimental objective: to study the inhibitory effects of the compound of formula (I) on the activity of 3CL protease derived from six other coronaviruses capable of infecting humans. The six viruses are SARS-CoV, MERS-CoV, H229E-CoV, HKU1-CoV, NL63-CoV, and OC43-CoV.

Materials:

3CL protease: A recombinant full-length coronavirus 3CL protease was prepared in-house according to the genome sequences of coronaviruses, and the genome GenBank numbers of the SARS-CoV, MERS-CoV, H229E-CoV, HKU1-CoV, NL63-CoV, and OC43-CoV used were AAP13442.1, MT387202.1, AF304460.1, AY597011.2, AY567487.2, and AY903459.1, respectively. The DNA sequences required for protein expression of the six coronavirus 3CL proteases were purchased from Nanjing GenScript Biotech Co., Ltd.

The 3CL protease substrate was purchased from Nanjing GenScript Biotech Co., Ltd.

The chymotrypsin substrate was purchased from GL Biochem.

Other reagents are shown in the table below:

| Reagent material | Brand | Catalog No. |
|---|---|---|
| Bovine pancreas-derived chymotrypsin | Sigma | C4129 |
| Tris | Sigma | BCBX3837 |
| EDTA | Sigma | 0001434776 |

Experimental procedures:

A reaction buffer (containing 50 mM Tris and 1 mM EDTA) was prepared. The test compound was dissolved in DMSO to obtain a 100 mM stock solution, which was further diluted in a 2-fold gradient with the reaction buffer to obtain a total of 11 concentrations. 3CL protease and compounds at different concentrations were added into a 96-well plate, and the plate was incubated at room temperature for 10 min. The substrate was then added, and the plate was promptly placed in a microplate reader for reading. The volume of the entire enzymatic reaction system was 120 μL, the final concentrations of the SARS-CoV, MERS-CoV, H229E-CoV, HKU1-CoV, NL63-CoV and OC43-CoV proteases were 30 nM, 80 nM, 30 nM, 20 nM, 30 nM and 10 nM, respectively, and the final concentration of the substrate was 10 μM. During the reading, the wavelengths of the excitation light and the emission light were 340 nm and 490 nm, respectively. The test duration was 10 min, with fluorescence readings taken every 1 min. The final results were taken from the readings obtained within the first 5 min for fitting to obtain the reaction rates. The inhibition rate was then calculated using the following formula: inhibition rate = 1 - (reaction rate of test group/reaction rate of control group).

Experimental results: As shown in Table 3, the compound of formula (I) exhibited a relatively good inhibitory effect on 3CL proteases derived from six other coronaviruses, suggesting that the compound of formula (I) may have a broad-spectrum anti-coronavirus activity.

**Table 3. Inhibitory effect of compound of formula (I) on 3CL proteases derived from other coronaviruses**

| 3CL protease source | Compound of formula (I) $IC_{50}$ (μM) |
|---|---|
| HKUl-CoV | 0.0049 |
| OC43-CoV | 0.010 |
| SARS-CoV | 0.024 |
| MERS-CoV | 0.060 |
| H229E-CoV | 0.13 |
| NL63-CoV | 0.85 |

**Test Example 3: Inhibitory Effect of Compound of Formula (I) on SARS-CoV-2 Vero E6 Prototype strain (WIV04), Delta Strain (B.1.617.2), and Omicron Strain (B.1.1.529) at Cell Level**

Experimental objective: In this experiment, the inhibitory effect of the compound of formula (I) on the replication of SARS-CoV-2 prototype strain (strain WIV04), Delta strain (B.1.617.2), and Omicron strain (B.1.1.529) in Vero E6 cells was investigated by real-time fluorescence quantitative PCR detection of the viral copy number in the culture supernatant. Since Vero E6 cells highly express the efflux transporter P-gp, 0.5 μM of the P-gp inhibitor CP-100356 was added for co-incubation with the compound.

Materials:

Vero E6 cells were purchased from ATCC (Cat. No. CRL-1586). The SARS-CoV-2 prototype strain (SARS-CoV-2-WIV04 strain), Delta strain (B.1.617.2), and Omicron strain (B.1.1.529) viruses were sourced from the Microorganisms and Viruses Culture Collection Center, Wuhan Institute of Virology, Chinese Academy of Sciences.

Other reagents are shown in the table below:

| Reagent name | Brand | Catalog No. |
|---|---|---|
| TaKaRa MiniBEST Viral RNA/DNA Extraction Kit Ver.5.0 | Takara | 9766 |
| TaKaRa PrimeScript™ RT reagent Kit with gDNA Eraser | Takara | RR047A |
| TaKaRa SYBR® Premix Ex Taq ™ II | Takara | RR820A |
| Fetal bovine serum | Gibco | R2768 |
| DMEM medium | Gibco | C11995500BT |
| CCK8 | Beyotime | C0039 |
| Chloroquine phosphate | SIGMA | C6628-50G |
| Trypsin | BIOSHARP | BL512A |

Experimental instrument:

Biosafety cabinet (AC2-3S1, ESCO, Singapore)
Carbon dioxide incubator (Thermo Scientific HERAcell 150i, Thermo Scientific, USA)
Water purifier (Shenyuan SYS ultra-pure water system, Chengdu)
StepOne Plus Real-time PCR system (4376600, ABI, USA)
TC20™ Automated Cell Counter (1450102, BIO-RAD, USA)
T100™ Thermal Cycler (1861096, BIO-RAD, USA)
Centrifuge (Micro21/21R Thermo Scientific, USA)

Experimental procedures:

Vero E6 cells were digested with trypsin, placed in a medium (90% DMEM, 10% fetal bovine serum), and seeded into a 48-well plate at 50000 cells/well, and the plate was incubated overnight. The test compound was dissolved in DMSO to prepare a 40 mM stock solution, which was further diluted in a gradient with a medium containing 0.5 μM of a Pgp inhibitor to obtain the concentration required for the test. The final concentration range of the test compound in the experiment was 1 μM to 0.004 μM. The cell supernatant was removed, the diluted compound (containing 0.5 μM of Pgp inhibitor) was added to each well, and the mixture was incubated for 1 h. Different strains of SARS-CoV-2 with a multiplicity of infection (MOI) of 0.01 or 0.001 were added to a biosafety level 3 (BSL-3) laboratory, and the mixture was incubated for 1 h. The supernatant was removed, and the plate was washed with PBS. The diluted compound (containing 0.5 μM of Pgp inhibitor) was added at 200 μL/well, and the supernatant was collected 24 h or 72 h after infection. The virus RNA in the supernatant was extracted and the virus copy number in the supernatant was detected using a real-time fluorescent quantitative PCR method. The inhibition rate of the compound was calculated based on the virus copy number, and the $IC_{50}$ of the compound was calculated using GraphPad Prism 8.

In the cytotoxicity assay, Vero E6 cells were digested, placed in a medium (90% DMEM, 10% fetal bovine serum), and seeded into a 96-well plate at 20000 cells/well, and the plate was incubated overnight. The test compound was dissolved in DMSO to prepare a 40 mM stock solution, which was further diluted in a gradient with a medium or a medium containing 0.5 μM of a Pgp inhibitor to obtain the concentration required for the test. The final concentration range of the test compound in the experiment was 500 μM to 1.95 μM. The cell supernatant was removed from the 96-well plate, and a medium containing the test compound (single-drug or containing 0.5 μM of the Pgp inhibitor) was added at 100 μL/well. After 24 h of incubation, the cell viability was measured using a CCK8 assay kit, and the inhibition rate and 50% cytotoxic concentration ($CC_{50}$) were calculated. Experimental results: As shown in Table 4, the compound of formula (I), when combined with the P-gp inhibitor CP-100356, could dose-dependently inhibit the replication of the Delta strain in Vero E6 cells, with an $IC_{50}$ value of 0.040 μM. The compound of formula (I) in combination with the P-gp inhibitor also exhibited a strong inhibitory effect in the prototype strain, with an $IC_{50}$ of 0.027 μM. In addition, the compound of formula (I) in combination with the P-gp inhibitor could significantly inhibit the replication of the Omicron strain in Vero E6 cells with an $IC_{50}$ of 0.12 μM. The compound of formula (I), single drug or in combination with the P-gp inhibitor, had no significant cytotoxicity on Vero E6 cell proliferation, with $CC_{50}$ > 500 μM.

**Table 4. Inhibitory effect of compound of formula (I) in combination with P-gp inhibitor on SARS-CoV-2 in Vero E6 cells**

| Experiment | Compound of formula (I) + 0.5 μM CP-100356 | |
|---|---|---|
| | $IC_{50}$ (μM) | Selection index SI SI=($CC_{50}$/$IC_{50}$) |
| Vero E6 prototype strain (WIV04) | 0.027 (mean, n = 2) | >18519 |
| Vero E6 Delta strain (B.1.617.2) | 0.040 (mean, n = 2) | >12500 |
| Vero E6 Omicron strain (B.1.1.529) | 0.12 | >4167 |
| Vero E6 single-drug $CC_{50}$ | >500 μM | |
| Vero E6 in combination with P-gp inhibitor $CC_{50}$ | >500 μM | |

**Test Example 4: *In Vivo* Antiviral Effect of Compound of Formula (I) on SARS-CoV-2 Delta Strain in hACE2-K18 Transgenic Mice**

Experimental objective: This study evaluated the antiviral activity of the compound of formula (I) against the SARS-CoV-2 Delta strain in K18 transgenic mice stably expressing human angiotensin-converting enzyme 2 (ACE2) (K18-hACE2).

Materials:

K18-hACE2 transgenic mice aged 7-8 weeks were purchased from Jiangsu GemPharmatech Co., Ltd. SARS-CoV-2 Delta strain virus was sourced from the Microorganisms and Viruses Culture Collection Center, Wuhan Institute of Virology, Chinese Academy of Sciences.

Ritonavir was purchased from Shanghai Desano Chemical Pharmaceutical Co., Ltd.

Vero E6 cells were purchased from ATCC (Cat. No. CRL-1586).

Other reagents are shown in the table below:

| Reagent name | Brand | Catalog No. |
|---|---|---|
| Qiagen 74106 RNeasy Mini Kit | Qiagen | 74106 |
| TaKaRa PrimeScript™ RT reagent Kit with gDNA Eraser | Takara | RR047A |
| TaKaRa SYBR® Premix Ex Taq ™ II | Takara | RR820A |
| Fetal Bovine Serum | Gibco | 10099-141C |
| DMEM medium | Gibco | C11995500BT |
| Tissue fixative solution | BOSTER | AR1068 |
| Aquacide II (sodium methylcellulose) | Millipore | 17851 |
| DMEM medium (powder, high sugar) | Gibco | 12100046 |
| Crystal violet | Sinopharm | 71012314 |

Experimental instrument:

Biosafety cabinet (AC2-3S1, ESCO, Singapore)
Carbon dioxide incubator (Thermo Scientific HERAcell 150i, Thermo Scientific, USA)
Water purifier (Shenyuan SYS ultra-pure water system, Chengdu)
StepOne Plus Real-time PCR system (4376600, ABI, USA)
TC20™ Automated Cell Counter (1450102, BIO-RAD, USA)
T100™ Thermal Cycler (1861096, BIO-RAD, USA)
Centrifuge (Micro21/21R Thermo Scientific, USA)
Tissue grinding instrument (JXFSTPRP-CL, Shanghai Jingxin, China)

Experimental procedures: K18-hACE2 transgenic mice were infected with SARS-CoV-2 Delta strain by nasal drop on day 0.2 h after infection, the vehicle and 50 mg/kg or 200 mg/kg of the compound of formula (I) (in combination with 50 mg/kg of the cytochrome P450 inhibitor ritonavir) were intragastrically administered BID for 2 days (wherein the administration was performed once on day 0, twice on day 1, and once on day 2) or 4 days (wherein the administration was performed once on day 0 and twice on days 1, 2, and 3). Changes in the body weight of the mice were recorded, and lung and brain tissues were collected at the endpoint. The left lung was fixed with formaldehyde and then subjected to embedding, sectioning and H&E staining for histopathological examination. The right lung and brain tissues were divided into two parts. One part was ground, and the homogenate was taken to extract RNA and carry out reverse transcription. The copy number of the virus was detected by real-time fluorescence quantitative PCR. The other part was ground, and the homogenate was taken for detecting the virus titer by a plaque assay. Plaque assay method: Vero E6 cells were seeded in a 24-well plate at 12000 cells per well and cultured overnight. The tissue homogenate stock solution was serially diluted 10-fold in DMEM medium for later use. The cell supernatant was removed, and the diluted tissue homogenate was added. The mixture was incubated for 1 h, and then the supernatant was removed. A medium containing 1% sodium methylcellulose and 2% FBS was added, and the mixture was cultured for 4 days. The medium was then removed, and after fixation with paraformaldehyde, the cells were stained with 1% (w/v) crystal violet, and the number of plaques in each well was counted.

Test results: As shown in Table 5, 2 days after infection, the compound of formula (I) at 50 mg/kg and 200 mg/kg in combination with ritonavir significantly reduced the viral load in the lungs compared with the model group (the average viral copy number was 9.19±0.30 log10 copies/g), and the average copy number was separately 7.66±0.27 log10 copies/g and 6.79±0.30 log10 copies/g, wherein the viral copy number was reduced by 2.4 log10 copies/g at the dose of 200 mg/kg. A persistent inhibition of viral copy number by the compound of formula (I) was observed after 4 days of infection.

In terms of the viral titer, as shown in FIG. 1, a significant inhibitory effect was observed for the compound of formula (I). 2 days after infection, the virus replication was completely inhibited at the dose of 200 mg/kg, and the titer was not detected. Compared to the model group, the viral titer was reduced by more than 3 log10 PFU/g at 50 mg/kg, and 4 days after infection, the compound of formula (I) showed a sustained inhibitory effect on the viral titer. The body weight is shown in FIG. 2. 4 days after infection, the body weight of the mice in the model group was reduced by about 10%, while the body weight of the administration group of the compound of formula (I) was not significantly reduced, indicating that the continuous administration of the compound of formula (I) showed no significant toxicity. We further detected the viral loads in the brains of the mice, and found no significant infections in the groups 2 days after infection. On day 4 after infection, the viral copy number in the brains of the mice was significantly reduced by the compound of formula (I) at both 50 mg/kg and 200 mg/kg as compared to the model group, and particularly, the viral copy number in the brains of the mice at the dose of 200 mg/kg was comparable to that of the uninfected normal group. We further detected the virus titer in the brains 4 days after infection, and the results are shown in FIG. 3. Compared with the model group, no virus titer was detected for the compound of formula (I) at both doses, indicating a strong inhibitory effect of the compound of formula (I). In addition, histopathological analysis of the lungs showed that compared with the model group, the compound of formula (I) at the dose of 200 mg/kg significantly ameliorated lung damage, including reducing the degree of alveolar atrophy or expansion and the degree of alveolar membrane thickening.

**Table 5. Viral loads in lungs and brains of mice 2 days and 4 days after infection (mean + SD)**

| Group | Lung tissue (log10 copies/g) | | Brain tissue (log10 copies/mg) | |
|---|---|---|---|---|
| | Day 2 | Day 4 | Day 2 | Day 4 |
| Model group | 9.19 ± 0.30 | 9.35 ± 0.30 | 1.34±0.78 | 7.17 ± 0.27 |
| Compound of formula (I) - 200 mg/kg + ritonavir - 50 mg/kg | 6.79 ± 0.30 | 7.48 ± 0.63 | 1.04±0.24 | 0.99 ± 0.42 |
| Compound of formula (I) - 50 mg/kg + ritonavir - 50 mg/kg | 7.66 ± 0.27 | 7.88 ± 0.74 | 0.77±0.20 | 2.88 ± 1.26 |
| Normal group | 5.76 ± 0.32 | 6.27 ± 0.18 | 1.08±0.06 | 1.08 ± 0.06 |

**Test Example 5: Selectivity of Compound of Formula (I) against Kinases**

Experimental objective: The inhibitory activity of the compound of formula (I) against 413 kinases was determined on the KinaseProfile experimental platform to study the selectivity of the compound of formula (I) against kinases.

Materials:

Full Human Panel [10 μM ATP] KinaseProfiler is a test product provided by Eurofins, Catalog No. 50-005KP10. This product contains 413 kinases.

Experimental procedures:

Each of the selected kinases was subjected to compound testing using Eurofins standard KinaseProfiler analytical method and following the relevant standard operating procedure. Protein kinases were detected by radiation method, while lipid kinases were detected by HTRF method.

The concentration of ATP in the experiment was 10 μM. Details of each kinase are available on the Eurofins website at the following address:
https://www.eurofinsdiscoveryservices.com/catalogmanagement/viewItem/Full-Human-Panel-10-uM-ATP-KinaseProfiler/50-005KP 10.

Experimental results: For 413 kinases, the inhibition rates of the compound of formula (I) were all less than 30% at the concentration of 10 μM, and no significant inhibitory effect was observed, suggesting that the compound of formula (I) has excellent selectivity.

**Test Example 6: Selectivity of Compound of Formula (I) against Safety Targets**

Experimental objective: The effect of the compound of formula (I) on 47 safety-related targets was detected on the Safetyscan experimental platform.

Materials:

Safety47 Panel Dose Response SAFETYscan is a test product provided by Eurofins, Cat. No. 87-1003DR. This product contains 78 tests related to 47 safety targets.

Experimental procedures:

For 78 tests related to 47 safe targets, the experimental methods used included: cAMP assay, calcium flux assay, hormone nuclear receptor assay, kinase binding assay, enzyme activity assay, neurotransmitter transporter assay, ion channel assay, and transporter assay. Specific methods for each experiment are available at the website of eurofins, which can be found at https://www.eurofinsdiscoveryservices.com/catalogmanagement/viewItem/Safety47-Panel-Dose-Response-SAFETYscan-DiscoverX/87-1003DR.

Experimental results: for 47 safety-related targets, the compound of formula (I) had no significant inhibition or activation effect ($EC_{50}$ was all greater than 100 μM) at the concentration of 100 μM, suggesting that the compound of formula (I) has excellent selectivity.

**Test Example 7: Human Plasma Protein Binding Assay of Compound of Formula (I)**

**Materials**

Human plasma was purchased from BioIVT, anticoagulated with EDTA K2, and stored at -80°C. The 96-well equilibrium dialysis plate was purchased from HTDialysis LLC. The equilibrium dialysis membrane was purchased from Gales Ferry.

**Experimental procedures**

An alkaline solution with a concentration of 14.2 g/L disodium hydrogen phosphate and 8.77 g/L sodium chloride was prepared with ultrapure water, and the alkaline solution could be stored at 4 °C for 7 days. An acidic solution with a concentration of 12.0 g/L sodium dihydrogen phosphate and 8.77 g/L sodium chloride was prepared with ultrapure water, and the acidic solution could be stored at 4 °C for 7 days. The alkaline solution was titrated with the acidic solution until the pH value was 7.4, and the buffer could be stored at 4 °C for 7 days. On the day of the experiment, the pH value of the buffer was measured, and if the pH value exceeded the range of 7.4±0.1, the pH value was adjusted.

The dialysis membrane was soaked in ultrapure water for 60 min to separate the membrane into two pieces, then soaked with 20% ethanol for 20 min, and finally soaked with dialysis buffer for 20 min.

The frozen plasma was quickly thawed at room temperature.

The plasma was then centrifuged at 4 °C for 10 min at a centrifugal force of 3,220 g to remove clots, and the supernatant was collected into a new centrifuge tube. The pH of the plasma was measured and recorded.

A 10 mM DMSO stock solution of the test substance was prepared. 2 μL of the stock solution (10 mM) was diluted with 98 μL of DMSO to give a working solution (200 μM). 3 μL of the working solution was taken, and 597 μL of human plasma was added to make a final concentration of 1 μM (0.5% DMSO). The mixture was vortexed to be mixed well.

120 μL of the plasma sample containing the compound was added to one side of the dialysis membrane, and a dialysate (phosphate-buffered saline) in the same volume was added to the other side. The experiment was conducted in duplicate. The dialysis plate was sealed, placed in an incubation device, and incubated at approximately 100 rpm at 37 °C with 5% $CO_2$ for 6 h. After the incubation was completed, the film was removed, and 50 μL was pipetted from the buffer side and the plasma side of each well to different wells of a new plate.

50 μL of blank plasma was added to the phosphate-buffered saline sample. A blank phosphate-buffered saline in the same volume was added to the plasma sample. 300 μL of room-temperature quencher (containing internal standard acetonitrile (IS, 500 nM labetalol, 100 nM alprazolam, and 2 μM ketoprofen)) was added to precipitate the protein. The mixture was vortexed for 5 min. The mixture was centrifuged at 3220 g at 4 °C for 30 min. 100 μL of the supernatant was transferred to a new plate. The supernatant was diluted with 100 μL or 200 μL of water based on the liquid chromatography-mass spectrometry response signal and the peak shape of the test substance. The mixture was well mixed, and the sample was analyzed using liquid chromatography-mass spectrometry.

All calculations were performed by Microsoft Excel. The peak areas of the test substance on the buffer side and the plasma side were determined. The formula for calculating the plasma protein binding rate of the test compound and the control drug is as follows: free rate = (ratio of sample peak area to internal standard peak area on buffer side/ratio of sample peak area to internal standard peak area on plasma side) × 100%, binding rate = 1 - free rate, recovery rate = (ratio of sample peak area to internal standard peak area on buffer side + ratio of sample peak area to internal standard peak area on plasma side)/(ratio of sample peak area to internal standard peak area in initial plasma sample) × 100%. The ratio of sample peak area to internal standard peak area on buffer side represents the free concentration of the compound, the ratio of sample peak area to internal standard peak area on plasma side represents the sum of the free concentration and binding concentration of the compound, and the ratio of sample peak area to internal standard peak area in initial plasma sample represents the total concentration of the compound at the initiation of sample incubation.

**Results:**

See Table 6. After incubation at 37 °C for 6 h, the compound of formula (I) at 1 μM showed an average free rate of 46.63%, a binding rate of 53.37%, and a recovery rate of 88.02%.

**Table 6. Results of human plasma protein binding assay of compound of formula (I)**

| Compound | Free rate (F%) | | | Binding rate (%) | Recovery rate (%) |
|---|---|---|---|---|---|
| | Replicate 1 | Replicate 2 | Mean value | | |
| Compound of formula (I) | 48.71 | 44.55 | 46.63 | 53.37 | 88.02 |

**Test Example 8: Tissue Distribution Assay of Compound of Formula (I) Following Single Intragastric Administration**

Materials:

60 Balb/c mice (purchased from Shanghai Minchang Biotechnology Co., Ltd.) were provided, half male and half female, weighing 18-25 g.

Experimental procedures:

Balb/c mice were given the compound of formula (I) by a single intragastric administration at a dose of 100 mg/kg and a volume of 10 mL/kg.

Before administration and 5 min, 0.25 h, 1.0 h, 2.0 h, 3.0 h, 5.0 h, 7.0 h and 10 h after administration (6 mice at each time point, half male and half female); 0.2 mL of blood was collected from the retrobulbar venous plexus at the time points set above, placed into an EDTA-K2 test tube, and centrifuged at 11000 rpm for 5 min. The plasma was separated and frozen in a refrigerator at -70 °C; at the time points of 0.25 h, 1.0 h, 3.0 h and 7.0 h, the whole blood was collected and the mice were immediately dissected to collect lung tissues. The tissues were washed with cold physiological saline to remove the residual blood and contents on the surface, dried, labeled and stored at -70 °C for later testing. The content of the

compound of formula (I) in plasma and lung tissue was determined by LC/MS-MS, and the lung/blood exposure ratio was calculated.

Results:

After a single intragastric administration of the compound of formula (I) to Balb/c mice, the ratio of lung tissue exposure to plasma exposure was 0.62, indicating that the compound of formula (I) had high exposure in lung tissues.

**Test Example 9: Safety Pharmacology Assay for Effect of Intragastric Administration of Compound of Formula (I) on Cardiovascular System in Cynomolgus Monkeys**

In the 2-week repeat-dose toxicity study in cynomolgus monkeys, the effect of the compound of formula (I) on the cardiovascular system was investigated concurrently.

Materials:

Thirty-two cynomolgus monkeys (half male and half female, aged 2.5-5 years) were used for administration.

Animal source: Yunnan Yingmao Biotechnology Co., Ltd.; Guangxi Xiongsen Primate Experimental Animal Breeding Development Co., Ltd.; Zhongke Lingrui (Zhanjiang) Biotechnology Co., Ltd.

Systolic blood pressure (SBP), diastolic blood pressure (DBP) and mean arterial pressure (MBP) of all conscious animals were measured using the intelligent non-invasive sphygmomanometer BP-98E using the Provantis/v10.2.3.1 electronic data capture system (PV-02).

Experimental procedures: Thirty-two cynomolgus monkeys were randomly grouped (group 1 and group 4: 5 animals/sex/group; group 2 and group 3: 3 animals/sex/group; 4 groups in total) and subjected to administration of the compound of formula (I) (40 mg/kg/day, 160 mg/kg/day, and 600 mg/kg/day) or the control formulation (98.9% vehicle formulation + 1.1% MTBE, 0 mg/kg/day) by nasogastric feeding twice daily for a total of 14 days, followed by a 14-day recovery period. All the animals were included in this study to evaluate the effects of the administration on ECG parameters (including heart rate, PR interval, QRS duration, QT interval and QTcF) and blood pressure in the pre-administration period, administration period and recovery period.

Experimental results: Under the conditions of this experiment, after the compound of formula (I) (40 mg/kg/day, 160 mg/kg/day, and 600 mg/kg/day) was intragastrically administered to the cynomolgus monkeys by nasogastric feeding twice daily for 14 days, no test sample-related cardiovascular system changes were observed; no test sample-related arrhythmia was observed; no test sample-related changes in ECG parameters or blood pressure were observed throughout the entire experiment.

**Test Example 10: Detection of Related Substances in Coated Tablets**

The related substances in formula F3 from Example 2 were determined.

The determination for related substances was performed according to high-performance liquid chromatography (General Chapter 0512, Chinese Pharmacopoeia, Volume IV, 2020 Edition), specifically as follows:

Test sample solution: Four tablets of the product were taken and added to a 500-mL volumetric flask, and a proper amount of water was added. The mixture was shaken with sonication to achieve disintegration, and then an equal volume of acetonitrile was added. The resulting mixture was sonicated for 15 min, then diluted to the volume with a solvent (water:acetonitrile = 1:1 v/v), thoroughly mixed by shaking, and centrifuged. A proper amount of the supernatant was precisely measured out and diluted with the solvent to prepare a solution containing about 1 mg of the compound of formula (I) per mL.

Control solution: A proper amount of the test sample solution was precisely measured out and quantitatively diluted with a solvent (water:acetonitrile = 1:1 v/v) to prepare a solution at a concentration of 10 μg/mL.

Chromatographic conditions: A linear gradient elution was performed using an octadecylsilane-bonded silica gel chromatographic column (Waters Atlantis T3, 4.6 mm × 150 mm, 3 μm) according to the following table, with the mobile phase A being a 0.01 mol/L ammonium perchlorate buffer (1.17 g of ammonium perchlorate was taken and dissolved in 1 L of water, and the mixture was adjusted to pH 2.5 with perchloric acid and thoroughly mixed), the mobile phase B being acetonitrile, the detection wavelength being 210 nm, the column temperature being 35 °C, the flow rate being 1.0 mL/min, and the sample injection volume being 10 μL.

| Time (min) | Mobile phase A (%) | Mobile phase B (%) |
|---|---|---|
| 0 | 80 | 20 |

(continued)

| Time (min) | Mobile phase A (%) | Mobile phase B (%) |
|---|---|---|
| 20 | 55 | 45 |
| 35 | 25 | 75 |
| 45 | 25 | 75 |
| 45.1 | 80 | 20 |
| 50 | 80 | 20 |

Determination method: The test sample solution and the control solution were precisely measured out and injected separately into a liquid chromatograph, and the chromatograms were recorded.

**Table 7. Determination results of related substances in coated tablets**

| Related substance (%) | Maximum individual impurity | Other individual impurities | Total impurities |
|---|---|---|---|
| | 0.05% | Undetectable | 0.05% |

The detection results of related substances in the coated tablets showed that the coated tablets obtained in the present disclosure had good quality and low contents of individual impurities and total impurities. Moreover, compared with the active pharmaceutical ingredient, the coated tablets of the present disclosure showed no increase in the maximum individual impurity and total impurities.

**Test Example 11: Dissolution Test of Coated Tablets**

The test was conducted according to the test method for dissolution and release rate (Method II, General Chapter 0931, Chinese Pharmacopoeia, Volume IV, 2020 Edition).

Using 900 mL of a 0.1 mol/L hydrochloric acid solution containing 0.5% sodium dodecyl sulfate as the dissolution medium, with a rotation speed of 75 rpm, the procedures were performed according to the method. Sampling was performed at 10 min, 15 min, 20 min, 30 min, and 45 min. Upon completion of the 45-min sampling, the rotation speed was adjusted to 200 rpm, and sampling was performed at 60 min.

Using 900 mL of a phosphate-buffered saline (pH 6.8) as the dissolution medium, with a rotation speed of 75 rpm, the procedures were performed according to the method. Sampling was performed at 5 min, 10 min, 15 min, 20 min, 30 min, 45 min, 60 min, 75 min, and 90 min. Upon completion of the 90-min sampling, the rotation speed was adjusted to 200 rpm, and sampling was performed at 120 min.

Using 900 mL of an acetate buffer (pH 4.5) as the dissolution medium, with a rotation speed of 75 rpm, the procedures were performed according to the method. Sampling was performed at 5 min, 10 min, 15 min, 20 min, 30 min, 45 min, 60 min, 75 min, and 90 min. Upon completion of the 90-min sampling, the rotation speed was adjusted to 200 rpm, and sampling was performed at 120 min.

The dissolution test results are shown in Table 8 and FIG. 4.

**Table 8. Dissolution test results of coated tablets**

| Dissolution medium | Time point (min) | 5 | 10 | 15 | 20 | 30 | 45 | 60 | 75 | 90 | 120 |
|---|---|---|---|---|---|---|---|---|---|---|---|
| 0.1 N hydrochloric acid + 0.5% SDS | Mean (%) | / | 59 | 72 | 79 | 88 | 94 | 96 | / | | |
| | RSD (%) | / | 3.7 | 2.2 | 1.6 | 1.1 | 0.8 | 0.9 | | | |
| Acetate buffer (pH 4.5) | Mean (%) | 32 | 56 | 67 | 74 | 84 | 91 | 94 | 97 | 98 | 99 |
| | RSD (%) | 7.4 | 1.6 | 1.2 | 1.1 | 1.2 | 1.6 | 1.0 | 1.3 | 1.1 | 1.4 |
| Phosphate-buffered saline (pH 6.8) | Mean (%) | 25 | 50 | 61 | 68 | 77 | 86 | 91 | 94 | 95 | 97 |
| | RSD (%) | 9.3 | 2.2 | 1.6 | 1.3 | 1.2 | 1.1 | 1.0 | 0.8 | 0.7 | 0.7 |

The dissolution test results in Table 8 and FIG. 4 showed that: in the 0.1 N hydrochloric acid + 0.5% SDS medium, acetate buffer (pH 4.5), and phosphate-buffered saline (pH 6.8), the coated tablets of the present disclosure exhibited good dissolution characteristics.

## Claims

1. An oral pharmaceutical composition, comprising a compound of formula (I) or a pharmaceutically acceptable salt thereof:

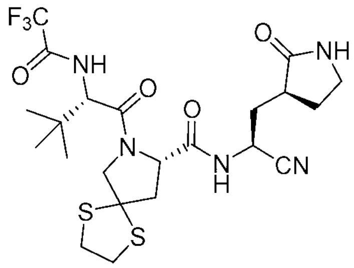

I

.

2. The oral pharmaceutical composition according to claim 1, wherein the oral pharmaceutical composition is in the form of a tablet, a capsule, a pill, a granule, a powder, an emulsion, a solution, or a suspension.

3. The oral pharmaceutical composition according to any one of claims 1-2, further comprising a disintegrant.

4. The oral pharmaceutical composition according to claim 3, wherein the disintegrant is selected from one or more of low-substituted hydroxypropyl cellulose, carboxymethylcellulose calcium, crospovidone, dry starch, sodium carboxymethyl starch, and croscarmellose sodium, or the disintegrant is selected from one or more of low-substituted hydroxypropyl cellulose, crospovidone, and croscarmellose sodium, or the disintegrant is croscarmellose sodium.

5. The oral pharmaceutical composition according to any one of claims 1-4, further comprising a filler.

6. The oral pharmaceutical composition according to claim 5, wherein the filler is selected from one or more of microcrystalline cellulose, calcium sulfate, calcium hydrogen phosphate, calcium carbonate, calcium sulfate dihydrate, lactose, sucrose, dextrin, sorbitol, starch or a derivative thereof, mannitol, xylitol, and fructose, or the filler is selected from one or more of microcrystalline cellulose, lactose, dextrin, and starch, or the filler is selected from one or more of microcrystalline cellulose and lactose, or the filler is microcrystalline cellulose and lactose.

7. The oral pharmaceutical composition according to any one of claims 1-6, further comprising a glidant.

8. The oral pharmaceutical composition according to claim 7, wherein the glidant is selected from one or more of colloidal silicon dioxide, talc, and wheat starch, or the glidant is selected from one or more of colloidal silicon dioxide, talc, and wheat starch, or the glidant is selected from one or more of colloidal silicon dioxide and talc, or the glidant is colloidal silicon dioxide.

9. The oral pharmaceutical composition according to any one of claims 1-8, further comprising a lubricant.

10. The oral pharmaceutical composition according to claim 9, wherein the lubricant is selected from one or more of magnesium stearate, stearic acid, calcium stearate, zinc stearate, sodium stearyl fumarate, liquid paraffin, polyethylene glycol, sodium dodecyl sulfate, and hydrogenated vegetable oil, or the lubricant is selected from one or more of magnesium stearate, stearic acid, calcium stearate, and sodium stearyl fumarate, or the lubricant is sodium stearyl fumarate.

11. The oral pharmaceutical composition according to any one of claims 1-10, comprising the compound of formula (I) or the pharmaceutically acceptable salt thereof, a disintegrant, a filler, a glidant, and a lubricant.

12. The oral pharmaceutical composition according to claim 11, wherein the disintegrant is croscarmellose sodium, the filler is microcrystalline cellulose and lactose monohydrate, the glidant is colloidal silicon dioxide, and the lubricant is sodium stearyl fumarate.

13. The oral pharmaceutical composition according to any one of claims 1-12, further comprising a coating agent, or further comprising a gastric-soluble film coating agent, wherein preferably, the coating agent comprises hydroxypropyl methylcellulose, iron oxide red, polyethylene glycol, and titanium dioxide.

**14.** A combination product, comprising: (1) the oral pharmaceutical composition according to any one of claims 1-13, and (2) another antiviral drug, or another anti-coronavirus and/or picornavirus drug, or ritonavir.

**15.** Use of the oral pharmaceutical composition according to any one of claims 1-13 or the combination product according to claim 14 in the preparation of a medicament for preventing or treating a related disease caused by infection with a coronavirus and/or a picornavirus.

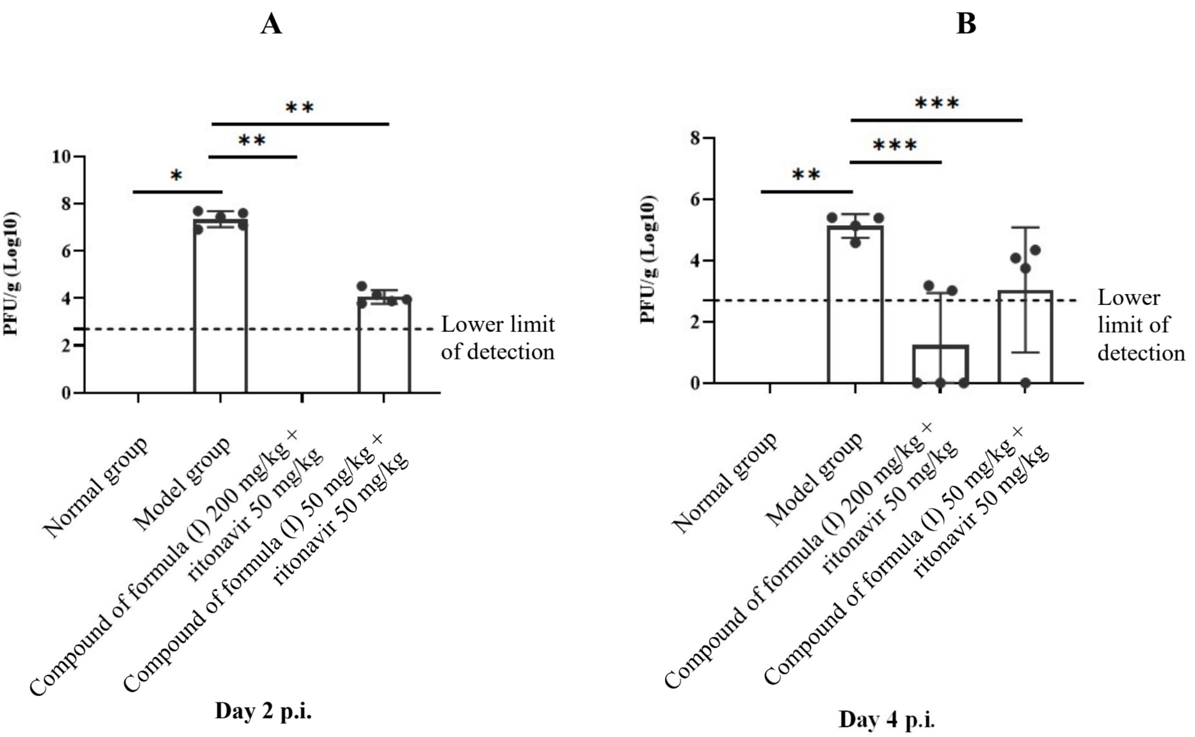
A
B
PFU/g (Log10)
Lower limit of detection
Normal group
Model group
Compound of formula (I) 200 mg/kg + ritonavir 50 mg/kg
Compound of formula (I) 50 mg/kg + ritonavir 50 mg/kg
Day 2 p.i.
Day 4 p.i.
*
**
***

FIG. 1

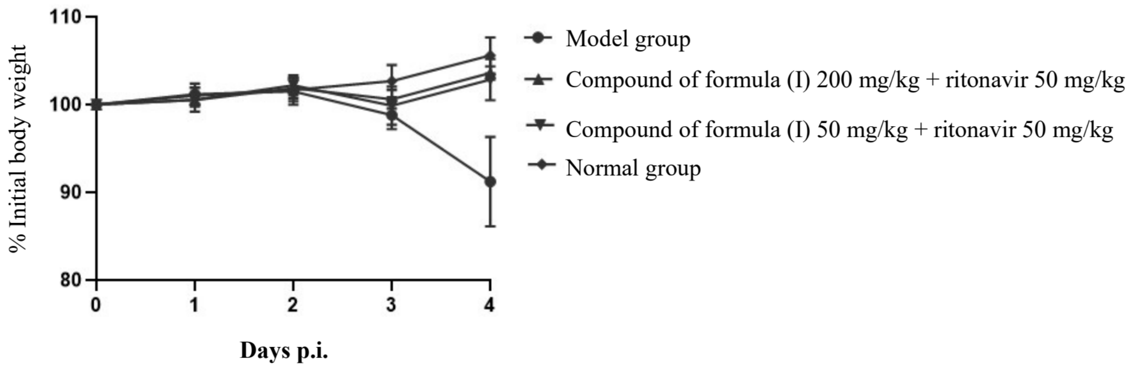
% Initial body weight
Days p.i.
Model group
Compound of formula (I) 200 mg/kg + ritonavir 50 mg/kg
Compound of formula (I) 50 mg/kg + ritonavir 50 mg/kg
Normal group

FIG. 2

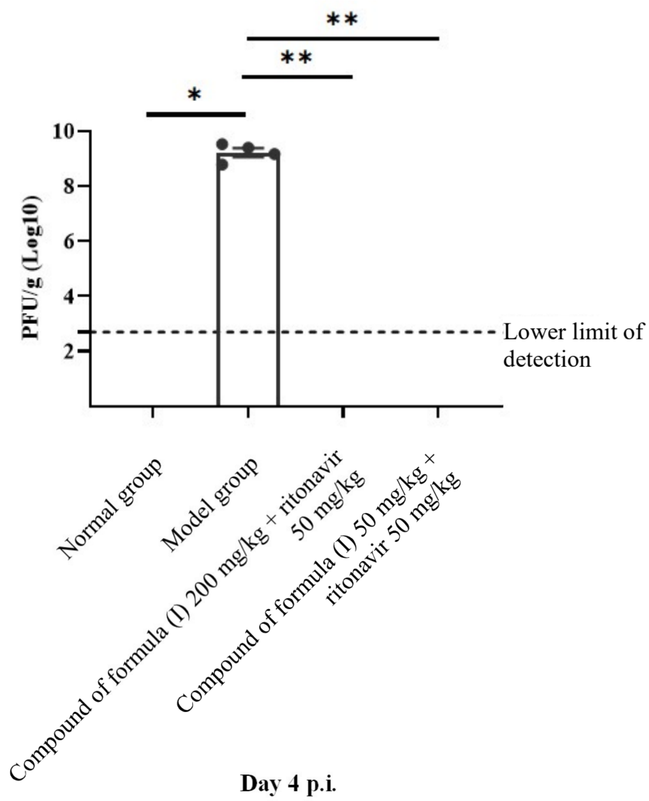
**
**
*
10
8
6
4
2
PFU/g (Log10)
Lower limit of detection
Normal group
Model group
Compound of formula (I) 200 mg/kg + ritonavir 50 mg/kg
Compound of formula (I) 50 mg/kg + ritonavir 50 mg/kg
Day 4 p.i.

FIG. 3

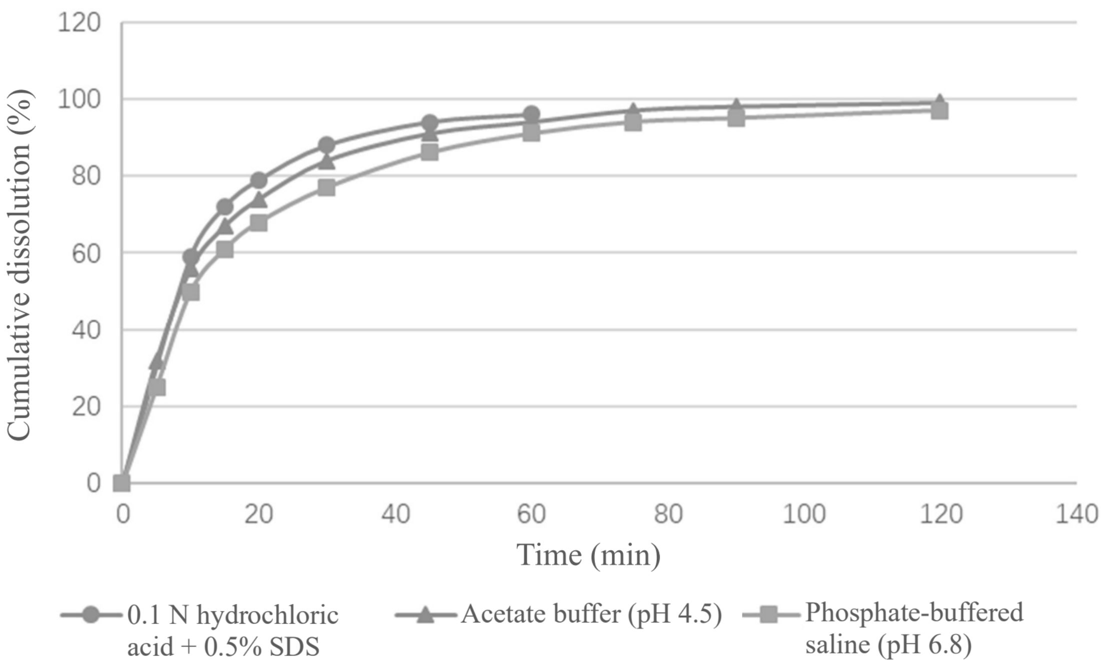
120
100
80
60
40
20
0
Cumulative dissolution (%)
0
20
40
60
80
100
120
140
Time (min)
0.1 N hydrochloric acid + 0.5% SDS
Acetate buffer (pH 4.5)
Phosphate-buffered saline (pH 6.8)

FIG. 4

## INTERNATIONAL SEARCH REPORT

International application No.
**PCT/CN2024/072261**

**A. CLASSIFICATION OF SUBJECT MATTER**

A61K31/407(2006.01)i; A61K31/427(2006.01)i; A61K9/48(2006.01)i; A61K9/28(2006.01)i; A61K9/20(2006.01)i; A61K9/16(2006.01)i; A61K9/107(2006.01)i; A61K9/08(2006.01)i; A61K47/38(2006.01)i; A61K47/26(2006.01)i; A61K47/04(2006.01)i; A61K47/02(2006.01)i; A61P31/14(2006.01)i

According to International Patent Classification (IPC) or to both national classification and IPC

**B. FIELDS SEARCHED**

Minimum documentation searched (classification system followed by classification symbols)

IPC:A61K, A61P

Documentation searched other than minimum documentation to the extent that such documents are included in the fields searched

Electronic data base consulted during the international search (name of data base and, where practicable, search terms used)

CNTXT, CNABS, ENTXT, ENTXTC, VEN, CNKI, WOTXT, USTXT, ISI_Web of Science, Pubmed, REGISTRY, CAPLUS: 氰基, 吡咯烷酮, 先声药业, 冠状病毒, 3CL蛋白酶, 基于分子式I进行了结构检索, cyano, pyrrolidone, coronavirus, 3CL protease, structure search based on the formula I

**C. DOCUMENTS CONSIDERED TO BE RELEVANT**

| Category* | Citation of document, with indication, where appropriate, of the relevant passages | Relevant to claim No. |
|---|---|---|
| PX | CN 117209556 A (SHANGHAI INSTITUTE OF MATERIA MEDICA, CHINESE ACADEMY OF SCIENCES et al.) 12 December 2023 (2023-12-12) description, paragraphs [0065]-[0066], [0104]- [0105], [0108], [0113], and [0243]- [0255] | 1-15 |
| A | US 11123329 B1 (VICORE PHARMA AB) 21 September 2021 (2021-09-21) description, column 3, line 29-column 7, line 13 | 1-15 |
| A | US 11124497 B1 (PARDES BIOSCIENCES, INC.) 21 September 2021 (2021-09-21) description, column 2, line 16-column 5, line 18, compounds 400-401 in table 1 in column 209-211, column 391, line 37-column 393, line 50, and column 400, line 60-column 402, line 8 | 1-15 |
| A | CN 112778310 A (SHANGHAI INSTITUTE OF MATERIA MEDICA, CHINESE ACADEMY OF SCIENCES et al.) 11 May 2021 (2021-05-11) description, paragraphs [0010]-[0012], [0080], [0086]-[0089], [00112]-[0113], and [0116] | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 April 2024** | **26 April 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/072261**

**C. DOCUMENTS CONSIDERED TO BE RELEVANT**

| Category* | Citation of document, with indication, where appropriate, of the relevant passages | Relevant to claim No. |
|---|---|---|
| A | CN 113181339 A (SHANGHAI INSTITUTE OF MATERIA MEDICA, CHINESE ACADEMY OF SCIENCES) 30 July 2021 (2021-07-30)<br>claims 8-10 | 1-15 |
| PX | JIANG, Xiangrui et al. "Structure-based development and preclinical evaluation of the SARS-CoV-2 3C-like protease inhibitor simnotrelvir"<br>*Nature Communications,* Vol. 14, 13 October 2023 (2023-10-13), 66463, and pages 1-15<br>ISSN: 2041-1723,<br>abstract, and page 3, figure 1 | 1-15 |

Form PCT/ISA/210 (second sheet) (July 2022)

**INTERNATIONAL SEARCH REPORT**
**Information on patent family members**

International application No.
**PCT/CN2024/072261**

| Patent document cited in search report | Publication date (day/month/year) | Patent family member(s) | Publication date (day/month/year) |
|---|---|---|---|
| CN 117209556 A | 12 December 2023 | CA 3230035 A1 | 06 April 2023 |
| | | WO 2023051657 A1 | 06 April 2023 |
| | | CN 116113631 A | 12 May 2023 |
| | | CN 116113631 B | 26 September 2023 |
| | | TW 202328145 A | 16 July 2023 |
| | | CN 117209555 A | 12 December 2023 |
| US 11123329 B1 | 21 September 2021 | EP 4125881 A1 | 08 February 2023 |
| | | WO 2021191592 A1 | 30 September 2021 |
| | | CA 3176108 A1 | 30 September 2021 |
| | | JP 2023520323 A | 17 May 2023 |
| | | US 2021290596 A1 | 23 September 2021 |
| | | US 2021386712 A1 | 16 December 2021 |
| US 11124497 B1 | 21 September 2021 | US 2022324844 A1 | 13 October 2022 |
| | | US 2021355111 A1 | 18 November 2021 |
| | | US 11312704 B2 | 26 April 2022 |
| | | US 2022402896 A1 | 22 December 2022 |
| | | US 2022162194 A1 | 26 May 2022 |
| | | US 11472793 B2 | 18 October 2022 |
| | | US 2023192663 A1 | 22 June 2023 |
| CN 112778310 A | 11 May 2021 | CN 112778310 A8 | 30 July 2021 |
| | | CA 3171091 A1 | 28 October 2021 |
| | | WO 2021213288 A1 | 28 October 2021 |
| | | CN 114096543 A | 25 February 2022 |
| | | CN 114096543 B | 16 August 2022 |
| | | AU 2021260618 A1 | 06 October 2022 |
| | | KR 20220143919 A | 25 October 2022 |
| | | AU 2021260618 A8 | 27 October 2022 |
| | | IL 297410 A | 01 December 2022 |
| | | BR 112022021226 A2 | 06 December 2022 |
| | | IN 202227061975 A | 09 December 2022 |
| | | MX 2022013270 A1 | 05 January 2023 |
| | | EP 4141007 A1 | 01 March 2023 |
| | | JP 2023526179 W | 21 June 2023 |
| | | US 2023219993 A1 | 13 July 2023 |
| | | US 11919923 B2 | 05 March 2024 |
| CN 113181339 A | 30 July 2021 | WO 2021151265 A1 | 05 August 2021 |
| | | CN 113181339 B | 22 February 2022 |
| | | CN 115087653 B | 20 September 2022 |
| | | EP 4098653 A1 | 07 December 2022 |
| | | US 2023138310 A1 | 04 May 2023 |
| | | CN 115087653 B | 17 November 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310081244


### Non-patent literature cited in the description


- Chinese Pharmacopoeia. 2020, vol. IV